# EUROPÄISCHE PATENTSCHRIFT

(11) **EP 3 456 374 B1**
(45) Veröffentlichungstag und Bekanntmachung des Hinweises auf die Patenterteilung: **20.11.2019**
(21) Anmeldenummer: 18000649.6
(22) Anmeldetag: 03.08.2018
(51) Int. Cl.: A61M 37/00, A61M 5/32, A61K 9/00

(54) **IMPLANTATSPRITZE**
IMPLANT SYRINGE
SERINGUE POUR IMPLANT

(30) Priorität: 19.08.2017 DE 102017007893
(43) Veröffentlichungstag der Anmeldung: 20.03.2019
(73) Patentinhaber: Gaplast Gmbh, 82442 Altenau (DE)
(72) Erfinder: Kneer, Roland, 82490 Farchant (DE); Kneer, Stephan, 82490 Farchant (DE)
(74) Vertreter: Flosdorff, Jürgen

(56) Entgegenhaltungen:
- DE-A1- 19 961 197
- DE-A1-102011 116 973
- DE-C1- 19 734 385
- DE-T2- 69 505 835
- DE-T2- 69 738 048
- US-A- 4 820 267

## Beschreibung

Die Erfindung betrifft eine Implantatspritze, die dazu verwendet wird, ein strangförmiges Präparat mit einem Langzeitwirkstoff in den Körper eines Patienten abzugeben. Meist wird das Langzeitpräparat in die Bauchdecke eines Patienten gelegt, in die zuvor mittels einer Spritzennadel ein Aufnahmekanal für das Präparat eingestochen wurde.

Aus der DE 197 34 385 C1 ist eine Implantatspritze bekannt, die eine Spritzennadelanordnung aus einer Spritzennadel, einem abstehenden Griffstück und einem Präparataufnahmeteil enthält, die miteinander verbunden sind und zur gemeinsamen axialen Bewegung in einer Führungseinrichtung sitzen. Die Implantatspritze hat ferner einen Kolben, dessen Vorschub in Richtung der Spritzennadel durch einen Anschlag so begrenzt ist, dass zwischen der Spitze der Spritzennadel und dem Kopfende des Kolbens ein Abstand verbleibt, der mindestens gleich der Länge eines abzugebenden Präparats ist, wobei die Spritzennadelanordnung mittels des Griffstücks mindestens um eine mit der Länge des Präparats übereinstimmende Strecke zurück ziehbar ist. Diese Implantatspritze wird so gehandhabt, dass zunächst der Einstichkanal ausgebildet wird, in dem die Spritzennadel soweit beispielsweise in die Bauchdecke eines Patienten eingestochen wird, bis die vordere Stirnkante einer Führungshülse der Implantatspritze auf der Haut des Patienten aufliegt. Anschließend wird der Kolben vorgeschoben, indem eine Basisplatte eines Abstandhalters der Implantatspritze hintergriffen und Druck auf eine mit dem Kolben verbundene Endplatte ausgeübt wird. Hierbei schiebt der Kolben das Präparat in die Spritzennadel vor, bis eine mit dem Kolben verbundene Hülse auf die Basisplatte des Abstandhalters auftritt. Anschließend wird die Implantatspritze an dem vorderen Griffstück und der Basisplatte des Abstandhalters ergriffen, woraufhin das Griffstück bis zu einem Anschlag zurück gezogen wird. Beim Auftreffen auf den Anschlag ist die Spritzennadel vollständig von dem Präparat zurück gezogen, das hiermit in dem Einstichkanal abgelegt ist, woraufhin die Implantatspritze entfernt wird. Mit einer derartigen Implantatspritze läßt sich ein Präparat schonend in dem Einstichkanal ablegen. Der Nachteil dieser bekannten Implantatspritze besteht darin, dass zum Zurückziehen der Spritzennadel ein Umgreifen erforderlich ist, wodurch die Handhabung der Spritze erschwert ist.

Aus der DE 199 61 197 B4 ist eine Implantatspritze bekannt, bei der dieser Nachteil vermieden ist. Bei dieser Implantatspritze muss die Griffstellung nicht geändert werden. Auf den Vorschub des Kolbens erfolgt unmittelbar das Zurückziehen der Spritzennadel, so dass deren Handhabung erleichtert ist und einen geringeren Zeitaufwand erfordert. Nachteile dieser Implantatspritze sind darin zu sehen, dass das Griffstück, mit dem die Spritzennadelanordnung zurückgezogen wird, am vorderen Endbereich der Implantatspritze angeordnet ist, so dass bei der Handhabung der Implantatspritze die Finger der Hand des Arztes breit gespreizt werden müssen, und dass nach Zurückziehen der Spritzennadel wegen der geschlitzten Führungshülse die Gefahr besteht, sich an der Spritzennadel zu verletzen. Da außerdem das Präparat durch eine Engstelle in dem Kanal von dem Kolben vorgeschoben wird, kann es zu einem Abrieb bei dem Präparat oder gar zu dessen Bruch kommen.

Siehe ferner DE69505835T T2.

Der vorliegenden Erfindung liegt die Aufgabe zugrunde, eine Implantatspritze anzugeben, bei der wenigstens einer der obigen Nachteile vermieden ist.

Die Aufgabe wird erfindungsgemäß durch die Merkmale des Patentanspruchs 1 gelöst.

Vorteilhafte Ausgestaltung der Erfindung sind in den Unteransprüchen gekennzeichnet.

Die erfindungsgemäße Implantatspritze hat eine Spritzennadelhalterung, an der eine Spritzennadel befestigt ist, an die sich ein Präparataufnahmeteil anschließt, dessen Kanal in den Kanal der Spritzennadel übergeht. Die Spritzennadelhalterung ist mit einer Außenhülse der Implantatspritze verbunden, die an ihrem rückwärtigen Ende einen überstehenden Griffabschnitt hat, der als vorderer Griffabschnitt bezeichnet wird, wobei die Spritzennadel, die Spritzennadelhalterung und das Präparataufnahmeteil mit der Außenhülse zur gemeinsamen axialen Bewegung miteinander verbunden sind.

Die Außenhülse sitzt verschieblich auf einer Innenhülse, wobei die Außenhülse nicht-drehbar gegenüber der Innenhülse angeordnet ist. Die Innenhülse hat in einem Zwischenbereich mehrere in Umfangsrichtung voneinander beabstandete Schlitze, die von Stegen durchgriffen sind, die die Außenhülse mit der Spritzennadelhalterung verbinden. Die Schlitze erstrecken sich vorzugsweise über eine Länge, die mit dem Bewegungsbereich der Spritzennadelhalterung übereinstimmt.

Außerdem enthält die erfindungsgemäße Implantatspritze einen stabförmigen Kolben, der an einer vorzugsweise kreisförmigen Platte befestigt ist, die als rückwärtiger Griffabschnitt bei der Betätigung der Implantatspritze dient. Der Kolben ist über einen rückwärtigen Teil seiner Längserstreckung von einer Hülse umgeben, die verschieblich nicht-drehbar in die Innenhülse eingreift. Der Kolben ist so weit durch das Präparataufnahmeteil in die Spritzennadel vorschiebbar, dass zwischen der Spitze der Spritzennadel und dem Kopfende des Kolbens ein Abstand verbleibt, der etwa gleich der Länge des abzugebenden Präparats oder etwas größer ist. Der Vorschub des Kolbens wird dadurch begrenzt, dass der rückwärtige Griffabschnitt die rückwärtige Randkante der inneren Hülse erreicht. Bis dies geschieht, ist die Außenhülse in ihrer vorgeschobenen Ausgangsstellung, in der die Spritzennadel frei liegt, durch eine Blockiereinrichtung arretiert, so dass die Außenhülse mit der Spritzennadel nicht zurück gezogen werden kann. Die Blockiereinrichtung ist vorzugsweise an der Innenhülse ausgebildet und überragt diese radial nach außen, so dass sie das Zurückziehen der Außenhülse blockiert. Sie kann in dieser Lage beispielsweise an einer rückwärtigen Schulter der Außenhülse anliegen. Beim Vorschub der hinteren Hülse in ihre Endstellung wird die Blockiereinrichtung radial nach Innen gezogen, so dass die Bewegungsbahn der Außenhülse freigegen ist, und diese zusammen mit der Spritzennadel mittels des vorderen Griffstücks zurückgezogen werden kann.

Bei dieser Ausbildung ist der Abstand zwischen den beiden Griffstücken, die zur Betätigung der Implantatspritze ergriffen werden, erheblich reduziert, da sich das vordere Griffstück am Ende der lang gestreckten Außenhülse befindet. Hierdurch ist die Handhabung der Implantatspritze beträchtlich erleichtert, insbesondere für solche Personen, die kleine Hände haben.

Mit großem Vorteil wird vorgeschlagen, dass die Blockiereinrichtung ein aus der Innenhülse freigeschnittener Federarm ist, dessen Rasthaken in dem von der hinteren Hülse unbelasteten Zustand radial über die Innenhülse vorsteht und das Zurückziehen der Außenhülse blockiert. Die hintere Hülse kann einen axialen Schlitz aufweisen, in den der Federarm - relativ gesehen - hinein gleitet, wobei wenigstens eine schräge Fläche auf einen seitlichen Ansatz des Federarms oder direkt auf den Federarm aufläuft, die den Federarm radial nach Innen zieht und damit den Rasthaken aus der Bewegungsbahn der Außenhülse entfernt.

Dieser Vorgang ist erfolgt, wenn die hintere Hülse ihre vordere Endstellung erreicht hat, in der der hintere Griffabschnitt an die rückwärtige Randkante der inneren Hülse anschlägt.

Der Rückzug der Außenhülse ist vorzugsweise dann beendet, wenn die beiden Griffabschnitte aneinander anliegen. Bevorzugt ist, dass bei vollständig zurückgezogener Außenhülse der Kolben die Spitze der Spritzennadel überragt.

In einer weiteren vorteilhaften Ausgestaltung der Erfindung ragt ein Federarm in den Kanal des Präparataufnahmeteils hinein, der auf dem Präparat aufliegt und dieses fixiert, oder den Kanal im unbelasteten Zustand soweit verengt, dass der Durchgang des Präparats blockiert ist, so dass das Präparat nicht ungewollt aus der Implantatspritze austreten kann. Mit Vorteil ist vorgesehen, dass der Federarm flach in den Kanal ragt, vorzugsweise flach konvex gekrümmt. Wenn der Kolben das Präparat durch diese Engstelle vorschiebt, wird der Federarm nach Außen gedrückt, wobei dieser Vorgang so schonend ausfällt, dass das Präparat nicht abgeschabt wird oder gar brechen kann.

Außerdem wird mit Vorteil vorgeschlagen, dass die Innenhülse einen an den geschlitzten Bereich anschließenden Kopfabschnitt hat, der über seinen Umfang geschlossen ist und der die Spitze der Spritzennadel in deren zurückgezogener Endstellung überdeckt. Der Kopfabschnitt hat dabei eine bevorzugt sich nach vorne verjüngende Kegelstumpfform. Hierdurch ist sichergestellt, dass auch bei ungeschickter Handhabung der vom Patienten entfernten Implantatspritze keine Verletzungsgefahr durch die Spitze der Spritzennadel besteht.

Weitere Einzelheiten der Erfindung ergeben sich aus der nachfolgenden Beschreibung einer bevorzugten Ausführungsform der Implantatspritze sowie anhand der Zeichnungen. Dabei zeigen in unterschiedlichen Maßstäben:
- Figuren 1a bis 1c: verschiedene Darstellungen einer Ausführungsform der Implantatspritze;

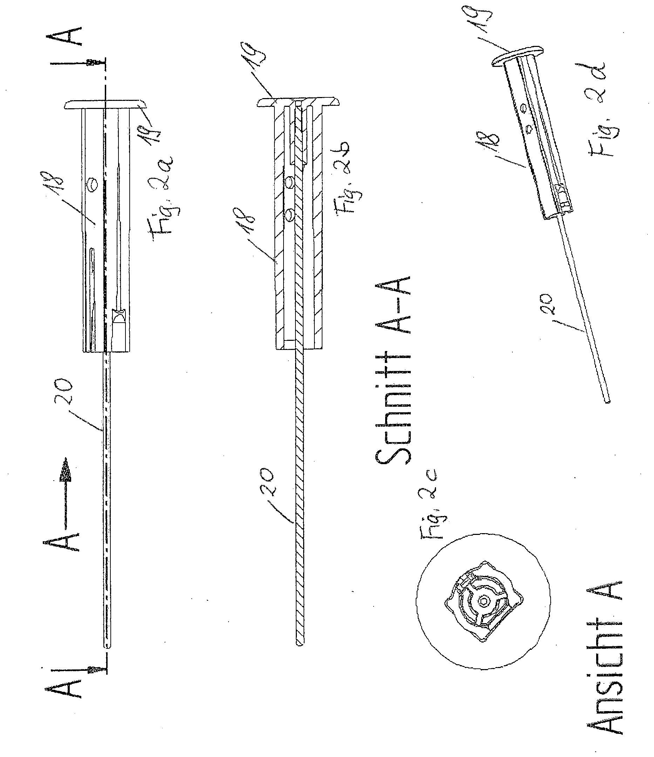

- Figuren 2a bis 2d: verschiedene Darstellungen der hinteren Hülse mit dem stabförmigen Kolben;

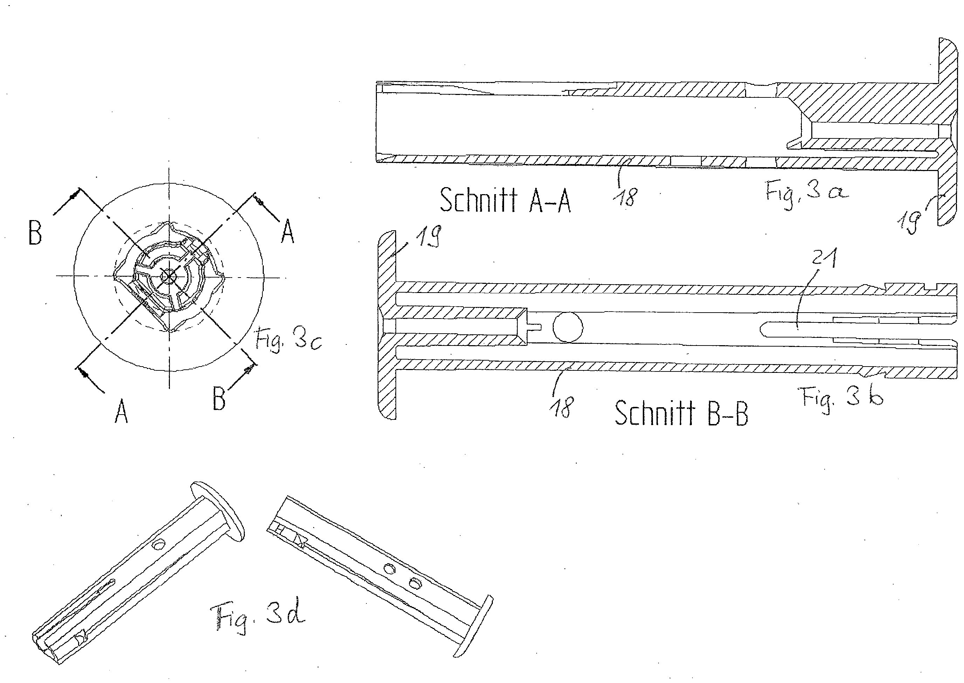

- Figuren 3a bis 3d: weitere Einzelheiten der hinteren Hülse;

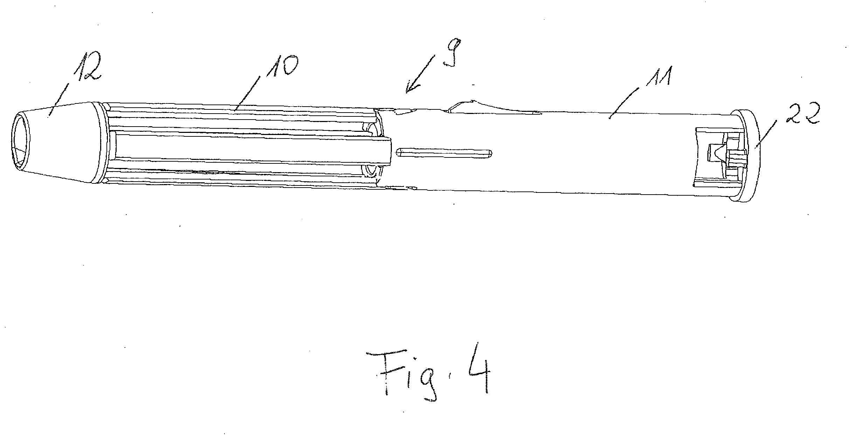

- Figur 4: eine Ansicht der aus zwei Teilen zusammen gesetzten Innenhülse;

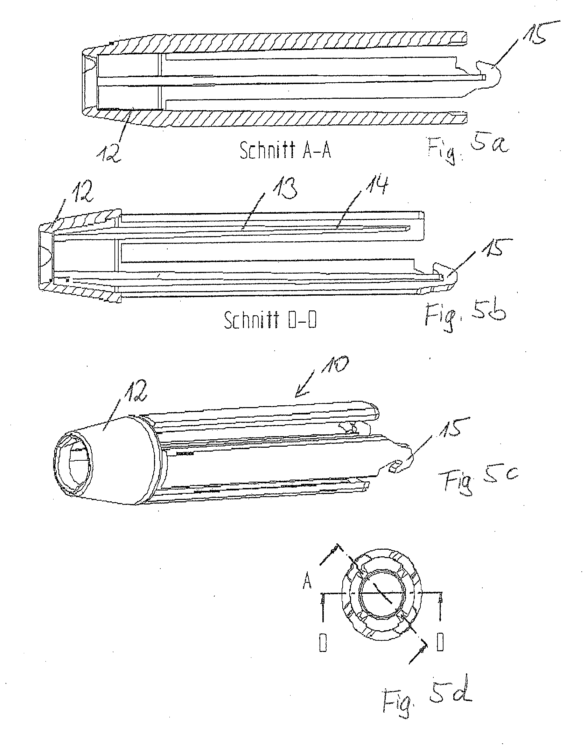

- Figuren 5a bis 5d: verschiedene Darstellungen des vorderen Teils der Innenhülse;

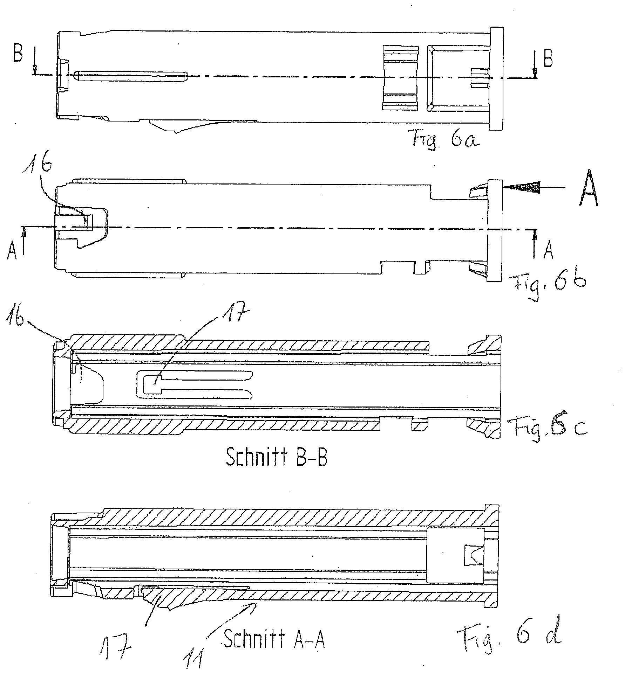

- Figuren 6a bis 6d: verschiedene Darstellungen des hinteren Teils der Innenhülse;

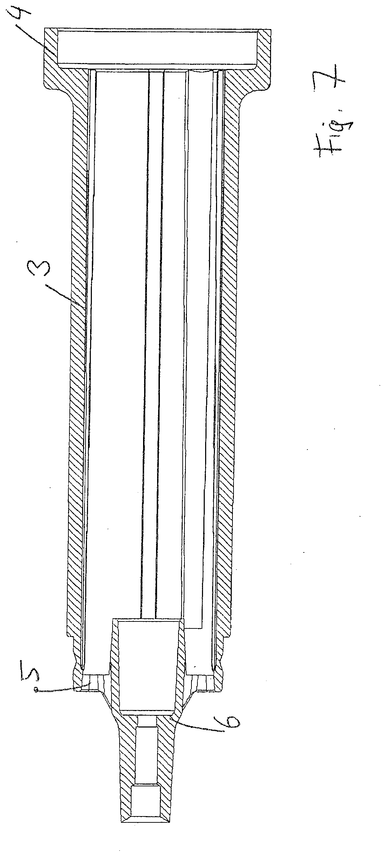

- Figur 7: einen Längsschnitt durch die Außenhülse;
- Figuren 8a bis 8d: verschiedene Darstellungen des Präparataufnahmeteils;
- Figur 9: die Blockiereinrichtung der Außenhülse.

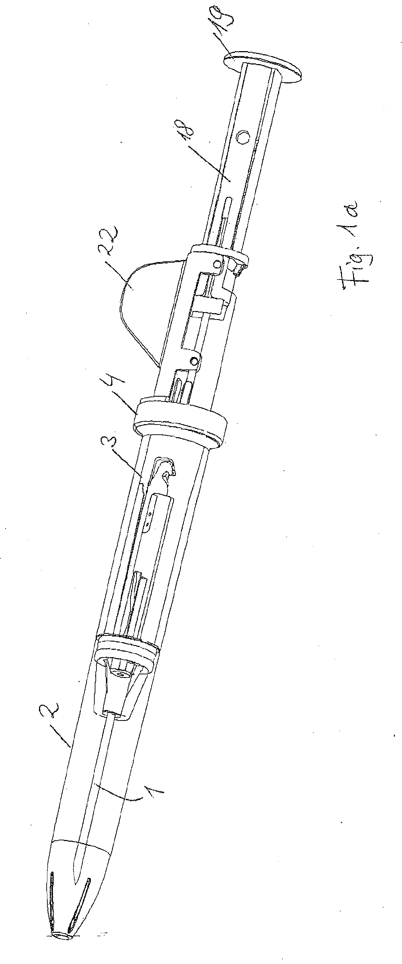

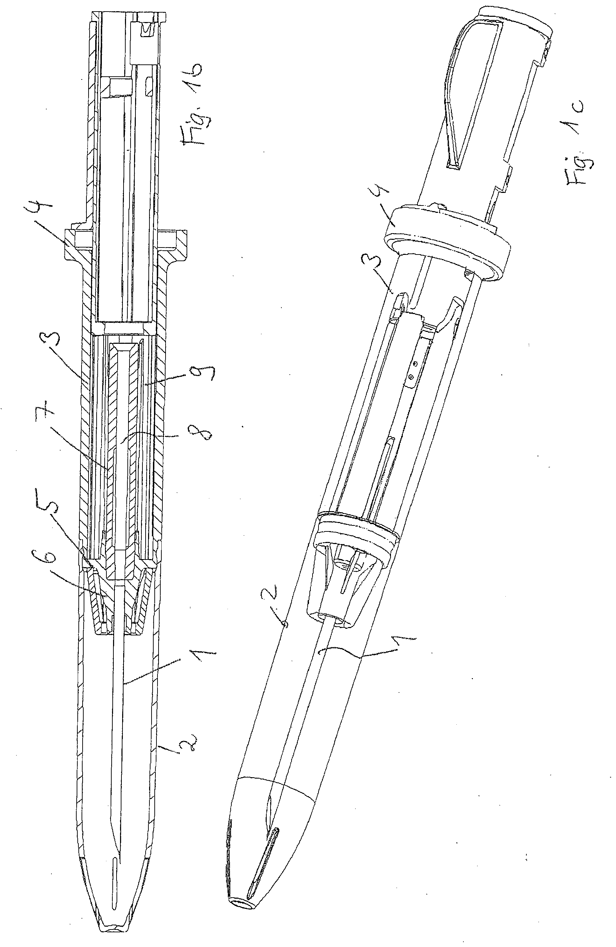

Die Figuren 1a bis 1c zeigen einen Ausgangszustand der Implantatspritze, bei dem die Spritzennadel 1 von einer abnehmbaren Schutzkappe 2 überdeckt ist. Die abnehmbare Schutzkappe 2 ist mit einer Außenhülse 3 leicht lösbar verrastet. Die Außenhülse 3 hat eine lang gestreckte zylindrische Form, wobei an ihrem in den Figuren rechten Endabschnitt ein radial überstehender vorderer Griffabschnitt 4 angeformt ist. Die Außenhülse 3 ist über mehrere radial nach Innen verlaufende Stege 5 mit einer Nadelhalterung 6 fest verbunden, in der die Spritzennadel 1 befestigt ist. Axial hinter der Nadelhalterung 6 ist ein Präparataufnahmeteil 7 so angeordnet, dass sein Kanal 8 mit dem Kanal der Spritzennadel 1 fluchtet. In diesem Präparataufnahmeteil 8 befindet sich ein nicht dargestelltes Präparat, das beim Gebrauch der Implantatspritze z.B. in der Bauchdecke eines Patienten abgelegt wird.

Die Außenhülse 3 sitzt (wenn sie nicht blockiert ist) verschieblich auf einer Innenhülse 9, die aus einem vorderen Teil 10 und einem hinteren Teil 11 zusammen gesetzt ist. Das vordere Teil 10 enthält einen vorderen, sich kegelstumpfförmig verengenden Abschnitt 12, der in Umfangsrichtung geschlossen ist und die Spitze der vollständig zurück gezogenen Spritzennadel 1 überdeckt. Der axial anschließnde Bereich enthält in Umfangsrichtung beabstandete, mit Schlitzen 14 versehende Stege 13, wobei an den Endabschnitten zweier Stege Haken 15 ausgebildet sind, die in Aussparungen 16 des hinteren Teil 11 der Innenhülse 9 einrasten, um die beiden Teile zu verbinden.

Der hintere Teil 11 der Innenhülse 9 enthält einen Federarm 17, der aus der Wand des Innenhülsenteils 11 frei geschnitten ist und mit einem Rasthaken 25 radial nach außen vorsteht. Dieser Federarm 17 mit dem Rasthaken 25 dient als Blockiereinrichtung für die Außenhülse 3, solange eine hintere Hülse 18 nicht in ihre Endstellung in den rückwärtigen Teil 11 der Innenhülse 9 eingeschoben ist.

An dem rückwärtigen Ende der hinteren Hülse 18 ist ein flaches Griffstück 19 angeformt, an dem mittig in der Hülse 18 ein stabförmiger Kolben 20 befestigt ist. Die hintere Hülse 18 enthält einen in axialer Richtung verlaufenden Schlitz 21, der beim Einschieben der hinteren Hülse 18 in den rückwärtigen Teil 11 der Innenhülse 9 mittels einer schrägen Fläche 26 so mit dem Federarm 17 zusammen wirkt, dass dessen Rasthaken 25 radial nach innen gezogen wird. Dies ist der Fall, wenn die Innenseite des hinteren Griffstücks 19 an der rückwärtigen Randkante 22 der Innenhülse 9 anschlägt, wobei diese Position leicht lösbar verrastet wird.

Nun kann die Außenhülse 3 zusammen mit der Spritzennadel 1 in ihrer Endposition zurück gezogen werden, in der die beiden Griffstücke 4 und 19 aneinander anliegen. Die Spitze der Spritzennadel 1 befindet sich nun innerhalb des kegelstumpfförmigen Abschnitts 12 der Innenhülse 9.

Figur 1a zeigt eine Sicherungshülse 22, die in dem Ausgangszustand der Implantatspritze mit Stegen den Vorschub der hinteren Hülse 18 und damit des Kolbens 20 blockiert. Die Sicherungshülse 22 wird vor Gebrauch der Implantatspritze abgenommen.

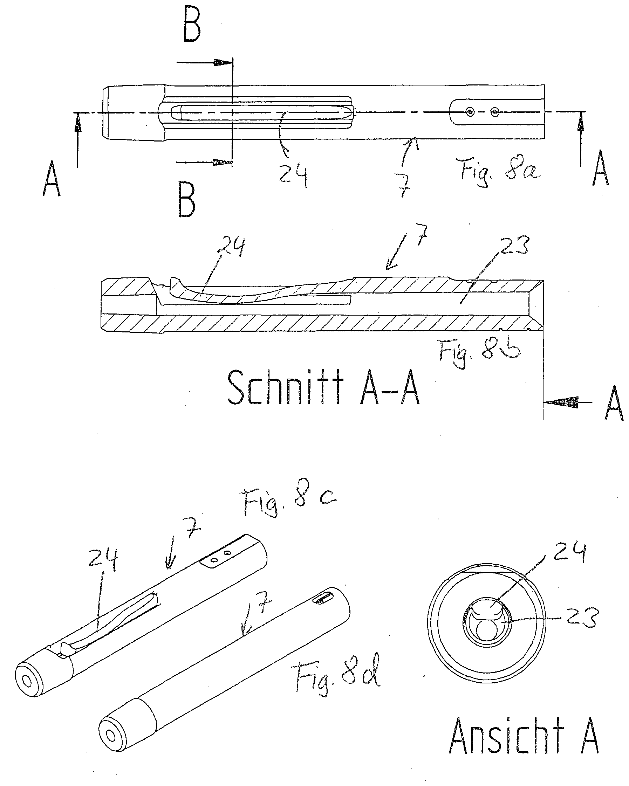

Die Figuren 8a bis 8d zeigen das Präparataufnahmeteil 7, das hinter der Spritzennadelhalterung angeordnet ist. In den Durchgangskanal 23 des Präparataufnahmeteils 7 ragt ein von der Umfangswand des Präparataufnahmeteils 7 freigeschnittener Federarm 24, der im unbelasteten Zustand den Durchgangskanal 23 so verengt, dass ein darin aufgenommenes Präparat den Federarm 24 nicht passieren kann. Der Federarm hat eine leicht konvex in den Kanal 23 hineinragende Form und ist einstückig mit dem übrigen Präparataufnahmeteil 7 ausgebildet. Wenn der Kolben 20 vorgeschoben wird, drückt er das Präparat glatt durch die Engstelle, wobei der Federarm etwas nach oben gedrückt wird. Hierdurch kann das Präparat nicht beschädigt werden.

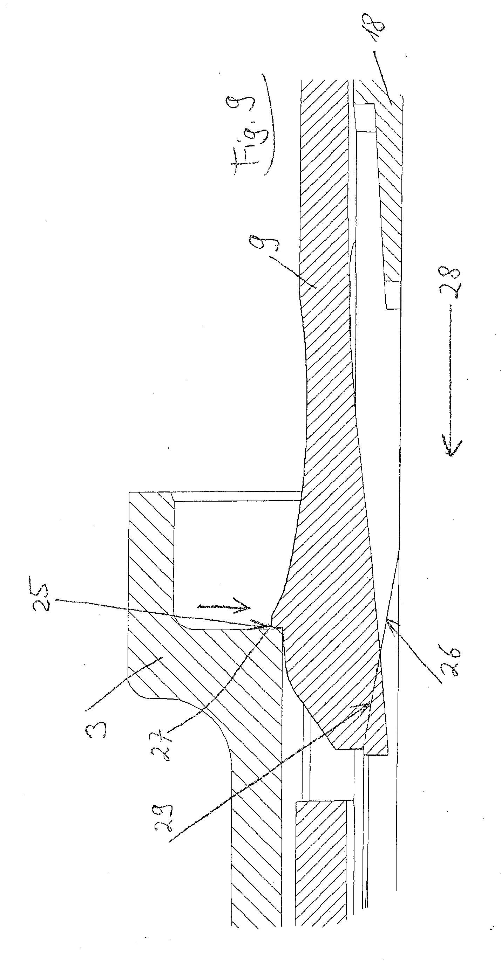

Figur 9 zeigt in einer vergrößerten schematischen Darstellung die Blockiereinrichtung für die Außenhülse 3. Die Innenhülse 9 liegt in dieser Darstellung mit einem radial nach außen überstehenden Rasthaken 25 an einer rückwärtigen Ringschulter 27 der Außenhülse an. Wenn die hintere Hülse 18 in Richtung des Pfeils 28 vorgeschoben wird, gleitet eine schräge Fläche 26 der hinteren Hülse 18 eine Auslöserampe 29 der Innenhülse 9 entlang, wodurch der Rasthaken 25 radial nach innen gezogen wird und den Rückzug der Außenhülse 3 frei gibt.

## Patentansprüche

1. Implantatspritze mit
einer Spritzennadelhalterung (6), die eine Spritzennadel (1) hält, mit einem axial anschließenden Präparataufnahmeteil (7) und mit einer Außenhülse (3) mit einem an ihrem rückwärtigen Endabschnitt radial überstehenden vorderen Griffabschnitt (4), wobei die Spritzennadelhalterung (6) und die Außenhülse (3) zur gemeinsamen axialen Bewegung miteinander verbunden sind, mit einer Innenhülse (9), auf der die Außenhülse (3) verschieblich sitzt und in der die Spritzennadel (1) mit dem Präparataufnahmeteil (7) verschieblich ist, wobei die Innenhülse (9) axiale Schlitze (14) aufweist, die Stege (5) durchgreifen, die die Außenhülse (3) mit der Spritzennadelhalterung (6) verbinden,
ferner mit einem stabförmigen Kolben (20), der an einem rückwärtigen Griffabschnitt (19) befestigt ist und teilweise von einer hinteren Hülse (18) umgeben ist, die verschieblich in die Innenhülse (9) eingreift,
wobei der stabförmige Kolben (20) durch das Präparataufnahmeteil (7) in die Spritzennadel (1) so weit vorschiebbar ist, dass zwischen der Spitze der Spritzennadel (1) und dem Kopfende des Kolbens (20) ein Abstand verbleibt, der etwa gleich der Länge des abzugebenden Präparats ist, und der Vorschub des Kolbens (20) dadurch begrenzt ist, dass der rückwärtige Griffabschnitt (19) die rückwärtige Randkante der Innenhülse (9) erreicht,
wobei die Außenhülse (3) in der vorgeschobenen Ausgangsstellung mit freiliegender Spritzennadel (1) durch eine Blockiereinrichtung (17), die an der Innenhülse (9) angeordnet ist und radial nach außen ragt, lösbar arretiert ist, und wobei die Blockiereinrichtung beim Vorschub der hinteren Hülse (18) in ihre Endstellung radial nach Innen bewegt wird, wodurch die Außenhülse (3) freigegeben ist und mittels des vorderen Griffabschnitts (4) auf der Innenhülse zurückziehbar ist.

2. Implantatspritze nach Anspruch 1,
**dadurch gekennzeichnet,**
**dass** die Blockiereinrichtung ein aus der Innenhülse freigeschnittener Federarm (17) ist, dessen vorzugsweise als Rasthaken (25) geformtes Kopfende im unbelasteten Zustand radial über die Innenhülse (9) vorsteht und das Zurückziehen der Außenhülse (3) blockiert.

3. Implantatspritze nach Anspruch 1 oder 2,
**dadurch gekennzeichnet,**
**dass** die hintere Hülse (18) einen axialen Schlitz aufweist, der über den Federarm (17) gleitet, der dabei durch wenigstens eine schräge Fläche (26) radial nach innen gezogen wird.

4. Implantatspritze nach einem der Ansprüche 1 bis 3,
**dadurch gekennzeichnet,**
**dass** die vordere Endstellung der hinteren Hülse (18) erreicht ist, wenn der rückwärtige Griffabschnitt (19) an die rückwärtige Randkante der Innenhülse (9) anschlägt.

5. Implantatspritze nach einem der Ansprüche 1 bis 4,
**dadurch gekennzeichnet,**
**dass** der Rückzug der Außenhülse (3) dadurch begrenzt ist, dass die beiden Griffabschnitte (4, 19) aneinander anliegen.

6. Implantatspritze nach einem der Ansprüche 1 bis 5,
**dadurch gekennzeichnet,**
**dass** bei vollständig zurückgezogener Außenhülse (3) der Kolben (20) die Spitze der Spritzennadel (1) überragt.

7. Implantatspritze nach einem der Ansprüche 1 bis 6,
**dadurch gekennzeichnet,**
**dass** in den Kanal (23) des Präparataufnahmeteils (7) ein Federarm (24) ragt, der im unbelasteten Zustand den Durchgang des Präparats blockiert.

8. Implantatspritze nach Anspruch 7,
**dadurch gekennzeichnet,**
**dass** der Federarm (24) flach konvex gekrümmt in den Kanal (23) ragt.

9. Implantatspritze nach einem der Ansprüche 1 bis 8,
**dadurch gekennzeichnet,**
**dass** die Innenhülse (9) einen an den geschlitzten Bereich anschließenden über den Umfang geschlossenen Kopfabschnitt (12) aufweist, der die Spitze der Spritzennadel (1) in deren zurück gezogener Endstellung überdeckt.

10. Implantatspritze nach Anspruch 9,
**dadurch gekennzeichnet,**
**dass** der Kopfabschnitt (12) eine sich nach vorne verjüngende Kegelstumpfform hat.

## Claims

1. An implant syringe with a syringe needle holder (6), which holds a syringe needle (1), an axially adjoining preparation receiving portion (7) and an outer sleeve (3) with a radially projecting handle section (4) on its rear end section, wherein the syringe needle holder (6) and the outer sleeve (3) are connected together for common axial movement, and with an inner sleeve (9), on which the outer sleeve (3) is slidably located and in which the syringe needle (1) is slidable with the preparation receiving portion (7), wherein the inner sleeve (9) affords axial slits (14), through which engage webs (5), which connect the outer sleeve (3) to the syringe needle holder (6), further including a rod-shaped piston, which is fastened to a rear handle section (19) and is partially surrounded by a rear sleeve (18), which slidably engages in the inner sleeve (9), wherein the rod-shaped piston (20) may be advanced so far through the preparation receiving portion (7) into the syringe needle (1) that a gap remains between the tip of the syringe needle (1) and the head end of the piston (20), which is approximately equal to the length of the preparation to be dispensed and the advance of the piston (20) is limited by the rear handle section (19) reaching the rear edge of the inner sleeve (9), wherein the outer sleeve (3) is releasably locked in the advanced starting position with the syringe needle (1) exposed by a blocking device (17), which is arranged in the inner sleeve (9) and projects radially outwardly, and wherein the blocking device is moved radially inwardly on advance of the rear sleeve (18) into its end position, whereby the outer sleeve (3) is released and is retractable by means of the front handle section (4) on the inner sleeve.

2. An implant syringe as claimed in Claim 1, **characterised in that** the blocking device is a spring arm (17) cut free from the inner sleeve, the head end of which, which is preferably shaped in the form of a locking hook (25), projects in the unloaded condition radially beyond the inner sleeve (9) and blocks the retraction of the outer sleeve (3).

3. An implant syringe as claimed in Claim 1 or 2, **characterised in that** the rear sleeve (18) affords an axial slot, which slides over the spring arm (17), which is thus drawn radially inwardly by at least one oblique surface (26).

4. An implant syringe as claimed in one of Claims 1 to 3, **characterised in that** the front end position of the rear sleeve (18) is reached when the rear handle section (19) abuts against the rear edge of the inner sleeve (9).

5. An implant syringe as claimed in one of Claims 1 to 4, **characterised in that** the return movement of the outer sleeve (3) is limited by the two handle sections (4, 19) engaging one another.

6. An implant syringe as claimed in one of Claims 1 to 5, **characterised in that** when the outer sleeve (3) is completely retracted, the piston (20) extends beyond the tip of the syringe needle (1).

7. An implant syringe as claimed in one of Claims 1 to 6, **characterised in that** projecting into the passage (23) of the preparation receiving portion (7) there is a spring arm (24), which in the unloaded state, blocks the passage of the preparation.

8. An implant syringe as claimed in Claim 7, **characterised in that** the spring arm (24) projects flat and convexly curved into the passage (23).

9. An implant syringe as claimed in one of Claims 1 to 8, **characterised in that** the inner sleeve (9) affords a head section (12), which adjoins the slitted region and is closed over its periphery and covers the tip of the syringe needle (1) in its retracted end position.

10. An implant syringe as claimed in Claim 9, **characterised in that** the head section (12) has a forwardly tapering frustoconical shape.

## Revendications

1. Seringue pour implant avec
une fixation d'aiguille de seringue (6), qui maintient une aiguille de seringue (1), avec une partie de réception de préparation (7) située dans le prolongement axialement et avec une douille extérieure (3) avec une section de préhension avant (4) dépassant radialement au niveau de sa section d'extrémité arrière, dans laquelle la fixation d'aiguille de seringue (6) et la douille extérieure (3) sont reliées l'une à l'autre aux fins du déplacement axial commun,
avec une douille intérieure (9), sur laquelle la douille extérieure (3) repose de manière à pouvoir coulisser et dans laquelle l'aiguille de seringue (1) peut coulisser avec la partie de réception de préparation (7), dans laquelle la douille intérieure (9) présente des entailles (14) axiales, qui traversent des nervures (5) qui relient la douille extérieure (3) à la fixation d'aiguille de seringue (6),
avec en outre un piston (20) en forme de barre, qui est fixé à une section de préhension arrière (19) et est entouré en partie d'une douille arrière (18), qui vient en prise de manière à pouvoir coulisser avec la douille intérieure (9),
dans laquelle le piston (20) en forme de barre peut être avancé par la partie de réception de préparation (7) dans l'aiguille de seringue (1) si loin qu'il reste, entre la pointe de l'aiguille de seringue (1) et l'extrémité de tête du piston (20), un espacement qui est environ égal à la longueur de la préparation à distribuer, et l'avancement du piston (20) est limité en ce que la section de préhension arrière (19) atteint l'arête de bord arrière de la douille intérieure (9),
dans laquelle la douille extérieure (3) est arrêtée de manière amovible, dans la position de départ avancée avec une aiguille de seringue (1) dégagée, par un dispositif de blocage (17), qui est disposé au niveau de la douille intérieure (9) et dépasse radialement vers l'extérieur, et dans laquelle le dispositif de blocage est déplacé, lors de l'avancement de la douille arrière (18) dans sa position finale, vers l'intérieur radialement, ce qui permet de dégager la douille extérieure (3) et de la rétracter au moyen de la section de préhension avant (4) sur la douille intérieure.

2. Seringue pour implant selon la revendication 1,
**caractérisée en ce**
**que** le dispositif de blocage est un bras de ressort (17) découpé de la douille intérieure, dont l'extrémité de tête mise en forme de préférence en tant que crochet d'enclenchement (25) fait saillie, dans l'état non soumis à une contrainte, radialement au-delà de la douille intérieure (9) et bloque la rétraction de la douille extérieure (3).

3. Seringue pour implant selon la revendication 1 ou 2,
**caractérisée en ce**
**que** la douille arrière (18) présente une entaille axiale, qui glisse sur le bras de ressort (17), qui est tiré radialement vers l'intérieur ce faisant par au moins une surface (26) oblique.

4. Seringue pour implant selon l'une quelconque des revendications 1 à 3,
**caractérisée en ce**
**que** la position finale avant de la douille arrière (18) est atteinte quand la section de préhension arrière (19) vient buter contre l'arête de bord arrière de la douille intérieure (9).

5. Seringue pour implant selon l'une quelconque des revendications 1 à 4,
**caractérisée en ce**
**que** le retrait de la douille extérieure (3) est limité **en ce que** les deux sections de préhension (4, 9) reposent l'une au niveau de l'autre.

6. Seringue pour implant selon l'une quelconque des revendications 1 à 5,
**caractérisée en ce**
**que**, lorsque la douille extérieure (3) est totalement rétractée, le piston (20) dépasse de la pointe de l'aiguille de seringue (1).

7. Seringue pour implant selon l'une quelconque des revendications 1 à 6,
**caractérisée en ce**
**que** dépasse dans le canal (23) de la partie de réception de préparation (7) un bras de ressort (24), qui bloque, dans l'état non soumis à une contrainte, le passage de la préparation.

8. Seringue pour implant selon la revendication 7,
**caractérisée en ce**
**que** le bras de ressort (24) dépasse dans le canal (23) avec une incurvation convexe plate.

9. Seringue pour implant selon l'une quelconque des revendications 1 à 8,
**caractérisée en ce**
**que** la douille intérieure (9) présente une section de tête (12), située dans le prolongement de la zone entaillée, fermée sur le pourtour, qui recouvre la pointe de l'aiguille de seringue (1) dans sa position finale rétractée.

10. Seringue pour implant selon la revendication. 9,
**caractérisée en ce**
**que** la section de tête (12) a une forme de cône tronqué se rétrécissant vers l'avant.
